# EUROPEAN PATENT APPLICATION

(11) **EP 2 692 337 A1**
(43) Date of publication of application: **05.02.2014**
(21) Application number: 12765686.6
(22) Date of filing: 27.03.2012
(51) Int. Cl.: A61K 8/73, A61K 8/02, A61K 8/42, A61K 9/70, A61K 47/38, A61Q 19/00

(54) **FILM-SHAPED EXTERNALLY USED DRUG COMPOSITION**

(30) Priority: 28.03.2011 JP 2011070529
(71) Applicant: Shiseido Company, Ltd., Tokyo 104-0061 (JP)
(72) Inventor: KISHIMOTO, Satoko, Yokohama-shi Kanagawa 224-8558 (JP); TAKASHIMIZU, Chisato, Yokohama-shi Kanagawa 224-8558 (JP); SAKATO, Yasuko, Yokohama-shi Kanagawa 224-8558 (JP); KITAMURA, Kenji, Yokohama-shi Kanagawa 224-8558 (JP)
(74) Representative: Henkel, Breuer & Partner
(86) International application number: PCT/JP2012/057934
(87) International publication number: WO 2012/133407

(57) **Abstract**

A film-shaped external preparation composition is provided, which is excellent in usability without occurrence of the residues due to a film-forming agent during or after use. The film-shaped external preparation composition comprises a water-soluble cellulose derivative as a primary component of the film; and contains hydroxyethyl urea. Also, it is preferred that the film-shaped external preparation composition is obtained by drying an aqueous solution comprising 40 to 70 mass% of the water-soluble cellulose derivative and 0.01 to 10 mass% of the hydroxyethyl urea. Also, it is preferred that, in the film-shaped external preparation composition, the water-soluble cellulose derivative is hydroxypropyl methylcellulose.

## Description

### RELATED APPLICATIONS

This application claims the priority of Japanese Patent Application No. 2011-070529 filed on March 28, 2011, which are incorporated herein by reference.

### FIELD OF THE INVENTION

The present invention relates to a film-shaped external preparation composition, particularly to usability improvement of the film-shaped external preparation composition.

### BACKGROUND OF THE INVENTION

As a method for providing cosmetic effects intensively on certain parts of the skin, a film-shaped external preparation obtained by adding an active ingredient to an aqueous solution of a film-forming agent such as a cellulose derivative, agar, gelatin and sugar; applying the resultant onto a substrate; and drying has been conventionally known. Usually, this type of external preparation is used by adding water to the preparation at an application site; dissolving the film; and packing or applying the dissolved film.

This type of film-shaped external preparation has an advantage of being able to provide components to an application site more efficiently and reliably than a sheet-type external preparation having a non-woven fabric impregnated with an active ingredient. In particular, a film-shaped external preparation having a cellulose derivative such as hydroxypropylethyl cellulose and methyl cellulose, which is used as a film-forming agent, has been reported to be excellent in solubility, hygroscopicity, desiccation resistance and blocking resistance (for example, Patent Literature 1: Japanese Unexamined Patent Application Publication No. 2007-326918).

### DISCLOSURE OF THE INVENTION

### PROBLEM TO BE SOLVED BY THE INVENTION

However, this type of film-shaped external preparation has a problem that a film-forming agent causes reformation of a film during or after application, resulting in occurrence of "residues" on the skin due to a manner of use in which the film was dissolved on the skin to apply an active ingredient not followed by subsequent washout.

Regarding this problem, occurrence of the residues has not been suppressed enough to solve difficulty in use.

The present invention was made in view of the above problem. Thus, an object of the present invention is to provide a film-shaped external preparation composition that is excellent in usability without occurrence of the residues due to a film-forming agent during or after use.

### MEANS TO SOLVE THE PROBLEM

The present inventors have conducted diligent studies to solve the above problems, and consequently completed the present invention by finding that occurrence of residues due to reformation of a film is prevented by blending hydroxyethyl urea in a film-shaped external preparation having a cellulose derivative as a film-forming agent.

Thus, a film-shaped external preparation composition comprises a water-soluble cellulose derivative as a primary component of the film; and contains hydroxyethyl urea.

Also, it is preferred that the film-shaped external preparation composition is obtained by drying an aqueous solution comprising 40 to 70 mass% of the water-soluble cellulose derivative and 0.01 to 10 mass% of the hydroxyethyl urea.

Also, it is preferred that, in the film-shaped external preparation composition, the water-soluble cellulose derivative is hydroxypropyl methylcellulose.

Also, it is preferred that the film-shaped external preparation composition is applied by adding water.

Also, it is preferred that the film-shaped external preparation composition is applied to the skin shortly after washing.

Also, a method of using the film-shaped external preparation composition comprises placing a gel sheet on the film-shaped external preparation to cover the composition.

### EFFECT OF THE INVENTION

The present invention can provide a film-shaped external preparation having a cellulose derivative that is less likely to generate residues when the cellulose derivative causes reformation of a film during or after an application. Therefore, a film-shaped product excellent in usability without making users uncomfortable can be provided in the fields of cosmetics and medicine.

### BEST MODE FOR CARRYING OUT THE INVENTION

The film-shaped external preparation composition according to the present invention contains a cellulose derivative as a film-forming agent as a primary component, blended with hydroxyethyl urea.

Generally, the cellulose derivative is a compound chemically modified at a part or all of hydroxyl groups of cellulose. In the present invention, a water-soluble cellulose derivative used especially in the fields of cosmetics and medicine can be used.

Examples of the cellulose derivative include water-soluble cellulose ethers such as methyl cellulose, hydroxyethyl cellulose, hydroxypropyl cellulose, hydroxypropyl methylcellulose, hydroxypropyl hydroxyethyl cellulose, hydroxyethyl methyl cellulose, ethyl cellulose and carboxymethyl cellulose. In the present invention, in particular, hydroxypropyl methylcellulose is preferably used.

The blending quantity of the cellulose derivative is preferably 40 to 70 mass%, and more preferably 55 to 65 mass% with respect to the composition. When the blending quantity of the cellulose derivative with respect to the total components used in manufacturing is less than 40 mass%, blocking resistance of the film can probably be insufficient.

In addition to the above cellulose derivatives, a publicly known film-forming agent used in the fields of cosmetics or medicine can be blended in the film-shaped external preparation composition according to the present invention.

Examples of the film-forming agent include polyvinyl alcohol, collagen, starch, gelatin, agar and sugar.

Examples of the starch, besides natural starch, include etherified (for example, hydroxyalkylated, such as hydroxypropylated, hydroxyethylated, hydroxymethylated and hydroxypropyl methylated) starches; esterified (for example, acetylated) starches; organic esterified starches and modified starches obtained as a product of a crosslinking reaction, oxidation, enzyme transformation and acid hydrolysis or the like of the above starches.

Examples of the sugar include a water-soluble polysaccharide such as pullulan, elsinan, sodium alginate, pectin, tamarind gum, xanthan gum, guar gum, tara gum, locust bean gum, arabinogalactan, gum arabic, chitosan, amylose, amylopectin, dextran and mannan.

A hydroxyethyl urea blended in the film-shaped external preparation composition according to the present invention is a compound represented by the structural formula below:

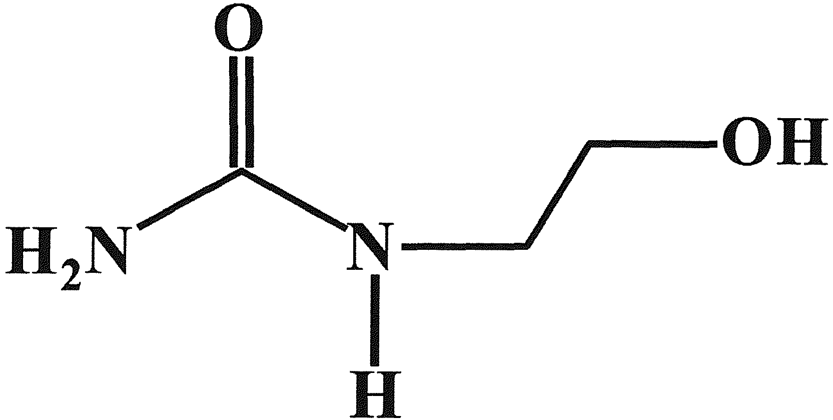

The hydroxyethyl urea having the above structural formula can be produced by a publicly known method such as a method in which monoethanol amine is reacted with urea in the presence of water. For stabilization of the produced hydroxyethyl urea, ammonia produced by the reaction is preferably neutralized with lactic acid or the like.

As a market product of the hydroxyethyl urea, for example, HYDROVANCE B of NSC Japan or the like is preferably used.

A hydroxyethyl urea content of the composition of the present invention is preferably 0.01 to 10 mass%, more preferably 0.01 to 5 mass%, and even more preferably 0.01 to 3 mass% with respect to the film-shaped external preparation composition.

When a blending quantity of hydroxyethyl urea in total components used in manufacturing is less than 0.01 mass%, residues due to reformation of the film are suppressed insufficiently; and when the blending quantity is more than 10 mass%, the composition can be felt sticky during use.

The film-shaped external preparation composition according to the present invention can be produced by applying and drying the aqueous solution of the above components on a substrate, and shaping the aqueous solution into a flat-sheet form. Specifically, the film-shaped external preparation composition can be formed by drying an aqueous solution preferably containing 40 to 70 mass% of a water-soluble cellulose derivative and 0.01 to 10 mass% of hydroxyethyl urea.

A thickness of the film-shaped external preparation composition after drying is preferably 5 to 15 µm, and more preferably 10 µm.

In addition to the above components, components generally used in cosmetics, medicines and non-medicinal products can be blended in the film-shaped external preparation composition according to the present invention so long as the effectiveness of the present invention is not undermined.

Examples of such components include humectants such as 1,3-butylene glycol, polyethylene glycol, propylene glycol, dipropylene glycol, hexylene glycol, glycerin, diglycerin, xylitol, maltitol, maltose, D-mannitol and hyaluronan; lower alcohols such as ethanol and propanol; ultraviolet absorbers such as benzoic acid-based ultraviolet absorbers (e.g. para-aminobenzoic acid), anthranilic acid-based ultraviolet absorber (e.g. methyl anthranilate), salicylic acid-based ultraviolet absorber (e.g. octyl salicylate and phenyl salicylate), cinnamic acid-based ultraviolet absorbers (e.g. isopropyl-p-methoxy cinnamate, octyl-p-methoxy cinnamate, and di-p-methoxycinnamic acid-mono-2-ethylhexanoic acid glyceryl), benzophenone-based ultraviolet absorbers (e.g. 2,4-dihydroxybenzophenone, 2-hydroxy-4-methoxybenzophenone, and 2-hydroxy-4-methoxybenzophenone-5-sulfonic acid), urocanic acid, 2-(2'-hydroxy-5'-methylphenyl) benzotriazol, 4-tert-butyl-4'-methoxybenzoylmethane; sequestrants such as edetate sodium, sodium metaphosphate and phosphoric acid; sequestrants such as ascorbic acid, alpha-tocopherol, dibutylhydroxytoluene, and butylhydroxyanisol; pH adjusters such as potassium hydroxide, citrate and acetate; chelators; preservatives such as paraben, phenoxyethanol, and chlorhexidine gluconate; fragrances; pigments; amino acids and amino-acid salts; inorganic salts such as sodium salt, calcium salt and potassium salt; powders such as talc, silica gel, zinc oxide, and titanium oxide. Especially among those above, a blending quantity of a moisturizer of a polyalcohol or the like in total components used in manufacturing of the film-shaped external preparation composition is preferably 30 mass% or less in the light of a film-forming of the cellulose derivative.

Also, oils such as hydrocarbon oils (e.g. vaseline, squalane and microcrystalline wax), ester oils (e.g. jojoba oil, spermaceti, and carnauba wax), triglycerides (e.g. olive oil and beef tallow), higher alcohols (e.g. cetanol, oleyl alcohol, and stearyl alcohol), higher fatty acids (e.g. stearic acid and oleic acid), silicone oils (e.g. dimethylpolysiloxane, decamethylcyclopentasiloxane, and polyether-modified silicone) and other oil-soluble components may be added by being emulsified or solubilized with surfactants.

Examples of surfactants include nonionic surfactants such as fatty acid monoglyceride and polyoxyethylene hydrogenated castor oil; anionic surfactants such as sodium lauryl sulfate and alkyl sulfosuccinate; cationic surfactants such as quaternary ammonium salts; ampholytic surfactants such as alkyl betaine; polymeric surfactants such as alkyl modified carboxyvinyl polymer.

Furthermore, various drugs can be blended according to the purpose in the film-shaped external preparation composition to provide drug efficacy.

Examples of the drug include vitamins such as vitamin A oil, retinol, retinol palmitate, inositol, pyridoxine hydrochloride, benzyl nicotinate, nicotinic acid amide, dl-α-tocopherol nicotinate, ascorbic acid magnesium phosphate, ascorbic acid-2-glucoside, vitamin D2 (ergocalciferol), potassium2-L-ascorbic acid diphosphate, dl-α-tocopherol, dl-α-tocopherol acetate, pantothenic acid, biotin, pyridoxine hydrochloride, CoQ10; an anti-inflammatory drug such as tranexamic acid, allantoin, glycyrrhizic acid salt, azulene, lysozyme chloride; a whitening agent such as arbutin and kojic acid; an astringent such as zinc oxide and tannin; sulphur; an α-lipoic acid; menthol: a tonic such as a γ-oryzanol, Korean ginseng and a sterol glycoside; an osmoregulating chemical; an antihistamine; a steroid hormone; a disinfectant; an antifungal agent; a vasoprotective drug; an antioxidant; a hypopigmentation drug; a desensitizer; an immunomodulator; an anti-aging drug; an anti-wrinkle agent; a sebum absorption agent; an antibiotic; a deodorant; and a fabric softener.

In addition, the above drugs can be used not only in a free form but also in form of a salt with an acid or a base when the drugs can form a salt in an ester form when the drugs have a carboxylic acid functional group.

In particular, the film-shaped external preparation composition according to the present invention is preferably applied to the skin shortly after washing. The skin shortly after washing refers to a naked skin having nothing applied thereon and shortly after washing. For example, when the present invention is used on the face, the film-shaped external preparation composition is applied after washing the face and before applying a basic skin-care product such as a lotion and a milk or a cosmetic material such as a foundation.

The film-shaped external preparation composition of the present invention can be used by adding water to the composition on the skin. A method of water addition is not especially limited; the water may be added to the film-shaped external preparation composition attached on skin; the film-shaped external preparation composition may be attached on a moist skin; or the water may be added to the film on a palm of a hand or the like and then the film may be applied to an application site. When the water is added to the film-shaped external preparation composition, water-soluble components constituting the film are gradually dissolved in water, and finally the film is completely dissolved to become a composition in a liquid or gel form. The composition in a liquid or gel form can supply its components to the skin, as necessary, by being applied onto an application site as required; being used as a pack by covering with a sheet material or the like; or being left on an application site as it is.

The water added to the film may be replaced by a liquid composition containing components except water, such as a lotion and an essence, as long as the liquid composition contains water in a sufficient amount to dissolve the film-shaped external preparation composition.

An amount of water added to the film is not particularly limited, but is preferably enough so that the film-shaped external preparation composition can be entirely moistened and the water-soluble components can be dissolved. Specifically, about 1 to 10% of water by volume of the film-shaped external preparation composition is preferably added.

A method for adding water and dissolving the film-shaped external preparation composition is not limited, but particularly, a gel sheet of a polysaccharide thickener is preferably used. The gel sheet refers to a gel sheet formed by gelling water with a polysaccharide thickener to obtain a composition; and forming the composition into a shape of a sheet. The gel sheet is usually used by covering on the film-shaped external preparation composition of the present invention attached on the skin. By such a manner of use, the film-shaped external preparation composition can be dissolved by water gradually released from the gel sheet. Furthermore, the dissolved external preparation composition can dissolve the film easily and efficiently by being kept sealed between the gel sheet and the skin.

As the gel sheet, for example, a gel sheet obtained by blending a composition containing 10 to 80 mass% of carrageenan, 5 to 50 mass% of locust bean gum, 5 to 50 mass% of xanthan gum and 5 to 50 mass% of mannan in an aqueous solution at a concentration of 1 to 6 mass% is preferably used. But the composition or the shape of the gel sheet is not limited so long as enough water is supplied to the film-shaped external preparation composition.

Furthermore, a state of dissolution of the film-shaped external preparation composition can be observed easily by using a semi-transparent or transparent gel sheet.

Application of the film-shaped external preparation composition according to the present invention is not particularly limited so long as an efficacy of the present invention is not undermined. But in particular, the film-shaped external preparation composition is preferably used as a film or a preparation for cosmetic and medical uses.

Examples of the product form of the present invention for cosmetic and medical uses include a moisturizing film, a film for improving rough skin, an anti-wrinkle film, a film for exfoliating, a film for skin pores, a moisturizing agent for body, a whitening agent for body, a sunscreen treatment agent, a film for wound or burn care, an antipruritic drug, a suppurative agent and an anti-inflammatory drug depending on components blended in the film-shaped external preparation composition.

In addition to the above forms for directly affecting a skin surface and its surrounding skin as described above, a form for targeting sites away from an application site by percutaneous administration of a drug or the like is also included.

The present invention is described further below by giving Examples, but is not limited by the Examples. A blending quantity is expressed by mass% with respect to a system to which the component is blended, unless otherwise specified.

### EXAMPLES

A practical use testing of a film-shaped external preparation composition of each Test Example produced from the compositions shown in Table 1 described below was conducted by 5 panels to score and evaluate easiness of dissolution (solubility) of a film and reduction of residues after use according to criteria described below. The results are shown in Table 1.

The method of use of the film-shaped external preparation composition of each example was as follows:

### <Method of use of the film-shaped external preparation composition>

The film (3 cm in size) was attached on the face soon after face wash. And then, a gel sheet having a composition described below was attached onto the film so that the film could be completely covered. When the film was completely dissolved with water released from the gel sheet (7 cm square), the gel sheet was removed and dissolved film components were spreaded on the skin with a hand. And then, a lotion and a milky lotion were normally used on a site to which the film components were applied.

### Criteria of solubility and residue reduction

### [Score Criteria]

5 points: Excellent
4 points: Good
3 points: Average
2 points: Poor
1 point: Extremely poor

### [Evaluation Criteria]

⊚: Total score of 5 panels were 20 points or more
○: Total score of 5 panels were 15 points or more and less than 20 points
Δ: Total score of 5 panels were 10 points or more and less than 15 points
×: Total score of 5 panels were less than 10 points

### Composition of gel sheet

| (Component) | (mass%) |
|---|---|
| (1) Locust bean gum | 0.5 |
| (2) Xanthane gum | 0.5 |
| (3) Mannan | 0.3 |
| (4) Carrageenan | 0.6 |
| (5) Glycerin | 5.0 |
| (6) 1,3-butylene glycol | 3.0 |
| (7) Dipropylene glycol | 5.0 |
| (8) PEG-60 hydrogenated castor oil | 0.3 |
| (9) Preservative | appropriate amount |
| (10) Fragrance | appropriate amount |
| (11) Water | Balance |

### (Manufacturing method of a gel sheet)

A mixture of (8) and (10) was added to an aqueous solution containing (11) in which (5) to (7) and (9) were dissolved, and stirred. And that, (1) to (4) were added to the aqueous solution and mixed to obtain a composition in a gel form, and the composition was formed into a shape of a sheet and cooled down.

**Table 1**

| | Test Example | | | | | |
|---|---|---|---|---|---|---|
| | 1-1 | 1-2 | 1-3 | 1-4 | 1-5 | 1-6 |
| Hydroxypropyl methylcellulose | 55.0 | 55.0 | 55.0 | 55.0 | 55.0 | 55.0 |
| Hydroxyethyl urea | -- | -- | -- | 0.01 | 1.5 | 10.0 |
| 1,3-butylene glycol | -- | 5.0 | 10.0 | 5.0 | 5.0 | 5.0 |
| Dipropylene glycol | -- | 5.0 | 8.0 | 5.0 | 5.0 | 5.0 |
| PEG-60 hydrogenated castor oil | 1.5 | 1.5 | 1.5 | 1.5 | 1.5 | 1.5 |
| Disodium edetate | Appropriate amount | Appropriate amount | Appropriate amount | Appropriate amount | Appropriate amount | Appropriate amount |
| Preservative | Appropriate amount | Appropriate amount | Appropriate amount | Appropriate amount | Appropriate amount | Appropriate amount |
| Water | Balance | Balance | Balance | Balance | Balance | Balance |
| Solubility | Δ | ○ | ⊚ | ⊚ | ⊚ | ⊚ |
| Residue reduction | Δ | Δ | Δ | ⊚ | ⊚ | ⊚ |

### (Manufacturing method of the water-soluble film)

Hydroxypropyl methylcellulose was added to water and mixed to obtain an aqueous solution. And then, the remaining components were added to the aqueous solution and mixed to obtain a homogeneous solution. The solution was applied on a substrate and dried to obtain a film-shaped composition 10 µm in thickness.

As shown in Test Examples 1-1 to 1-3, when polyalcohols (1,3-butylene glycol and dipropylene glycol) were blended in the film-shaped external preparation composition blended with hydroxypropyl methylcellulose as a film-forming agent, solubility was improved but occurrence of residues was not suppressed. On the other hand, in Test Examples 1-4 to 1-6 containing hydroxyethyl urea, solubility was improved without an increase in the amounts of the polyalcohols, and occurrence of residues was significantly suppressed. In further investigation, the improvements of solubility and the residue reduction were prominent especially when a blending quantity of hydroxyethyl urea was 0.01 to 5 mass%.

Therefore, in the present invention, hydroxyethyl urea is preferably blended at 0.01 to 10 mass%, and more preferably 0.01 to 5 mass%.

A practical use testing (5 panels) of a film-shaped external preparation composition produced from the composition represented in Table 2 below was conducted according to the following method of use and the residue reduction was evaluated according to the same criteria of the above test. The results are shown in Table 2.

### <Method of use A>

The film (3 cm in size) was attached on the face soon after face wash; and a gel sheet having the above composition was attached on the film so that the film could be completely covered. When the film was completely dissolved with water released from the gel sheet (7 cm square), the gel sheet was removed and dissolved film components were spreaded on skin with a hand. And then, a lotion and a milky lotion were normally used on a site to which the film components were applied.

### <Method of use B>

After face wash, a lotion and a milky lotion were normally used. And then, the film (3 cm in size) was attached on the face; and a gel sheet having the above composition was attached on the film so that the film can be completely covered. When the film was completely dissolved with water released from the gel sheet (7 cm square), the gel sheet was removed and dissolved film components were spreaded on the skin with a hand.

**Table 2**

| | Test Example | | | |
|---|---|---|---|---|
| | 2-1 | 2-2 | 2-3 | 2-4 |
| Hydroxypropyl methylcellulose | 60.0 | 60.0 | 60.0 | 60.0 |
| Hydroxyethyl urea | -- | 0.1 | -- | 0.1 |
| 1,3-butylene glycol | 10.0 | 10.0 | 10.0 | 10.0 |
| Dipropylene glycol | 8.0 | 8.0 | 8.0 | 5.0 |
| PEG-60 hydrogenated castor oil | 1.5 | 1.5 | 1.5 | 1.5 |
| Disodium edetate | Appropriate amount | Appropriate amount | Appropriate amount | Appropriate amount |
| Preservative | Appropriate amount | Appropriate amount | Appropriate amount | Appropriate amount |
| Water | Balance | Balance | Balance | Balance |
| Method of use | A | A | B | B |
| Residue reduction | Δ | ⊚ | × | Δ |

### (Manufacturing method of the water-soluble film)

Hydroxypropyl methylcellulose was added to water and mixed to obtain an aqueous solution. And then, the remaining components were added to the aqueous solution and mixed to obtain a homogeneous solution. The solution was applied on a substrate and dried to obtain a film-shaped composition 10 µm in thickness.

As shown in Table 2, in the method of use A in which the film-shaped external preparation composition was applied shortly after washing the face, an occurrence of residues resulting from reformation of a film by a film-forming agent was significantly suppressed by blending hydroxyethyl urea (Test Examples 2-1 and 2-2). On the other hand, in the method of B in which the film-shaped external preparation composition was applied after use of a lotion and a milky lotion, improvement by blending hydroxyethyl urea was shown but residues occurred even when the component was blended in.

Therefore, the film-shaped external preparation composition according to the present invention is preferably used on the skin shortly after washing.

Formulation Examples relating to manufacturing of the film-shaped external preparation composition of the present invention are shown below, but these are merely an example and do not limit the present invention. All blending quantities are shown as mass%.

### Formulation Example 1: Film for improving rough skin

| (Component) | (mass%) |
|---|---|
| Hydroxypropyl methylcellulose | 60 |
| Hydroxyethyl urea | 0.1 |
| 1,3-butylene glycol | 10 |
| Dipropylene glycol | 8 |
| PEG-60 hydrogenated castor oil | 1.5 |
| Tranexamic acid | 1 |
| Disodium edetate | appropriate amount |
| Preservative | appropriate amount |
| Water | Balance |

### (Manufacturing method)

Hydroxypropyl methylcellulose was added to water and mixed to obtain an aqueous solution. And then, the remaining components were added to the aqueous solution and mixed to obtain a homogeneous solution. The solution was applied on a substrate and dried to obtain a film for improving rough skin.

### Formulation Example 2: Film for improving rough skin

| (Component) | (mass%) |
|---|---|
| Hydroxypropyl methylcellulose | 60 |
| Hydroxyethyl urea | 0.1 |
| 1,3-butylene glycol | 10 |
| Dipropylene glycol | 8 |
| PEG-60 hydrogenated castor oil | 1.5 |
| Potassium 4-methoxysalicylate | 1 |
| Disodium edetate | appropriate amount |
| Preservative | appropriate amount |
| Water | Balance |

### (Manufacturing method)

Hydroxypropyl methylcellulose was added to water and mixed to obtain an aqueous solution. And then, the remaining components were added to the aqueous solution and mixed to obtain a homogeneous solution. The solution was applied on a substrate and dried to obtain a film for improving rough skin.

### Formulation Example 3: Film for skin pores

| (Component) | (mass%) |
|---|---|
| Hydroxypropyl methylcellulose | 60 |
| Hydroxyethyl urea | 0.1 |
| 1,3-butylene glycol | 10 |
| Dipropylene glycol | 8 |
| PEG-60 hydrogenated castor oil | 1.5 |
| Retinol | 1 |
| Disodium edetate | appropriate amount |
| Preservative | appropriate amount |
| Water | Balance |

### (Manufacturing method)

Hydroxypropyl methylcellulose was added to water and mixed to obtain an aqueous solution. And then, the remaining components were added to the aqueous solution and mixed to obtain a homogeneous solution. The solution was applied on a substrate and dried to obtain a film for skin pores.

## Claims

1. A film-shaped external preparation composition, comprising a water-soluble cellulose derivative as a primary component of the film; and hydroxyethyl urea.

2. The film-shaped external preparation composition according to claim 1, obtained by drying an aqueous solution comprising 40 to 70 mass% of the water-soluble cellulose derivative and 0.01 to 10 mass% of the hydroxyethyl urea.

3. The film-shaped external preparation composition according to claim 1 or 2, wherein the water-soluble cellulose derivative is hydroxypropyl methylcellulose.

4. The film-shaped external preparation composition according to any one of claims 1 to 3, being applied by adding water.

5. The film-shaped external preparation composition according to any one of claims 1 to 3, wherein the composition is applied to the skin shortly after washing.

6. A method of using the film-shaped external preparation composition, comprising:
placing a gel sheet on the film-shaped external preparation composition according to any one of claims 1 to 5 to cover the composition.
